# EUROPÄISCHE PATENTANMELDUNG

(11) **EP 0 837 059 A1**
(43) Veröffentlichungstag der Anmeldung: **22.04.1998**
(21) Anmeldenummer: 97117666.4
(22) Anmeldetag: 13.10.1997
(51) Int. Cl.: C07D 251/28

(54) **Cyanurchlorid-Formlinge und Verfahren zu ihrer Herstellung**

(30) Priorität: 15.10.1996 DE 19642449
(71) Anmelder: Degussa Aktiengesellschaft, 60311 Frankfurt (DE)
(72) Erfinder: Goedecke, Ralf, Dr., 63417 Rodenbach (DE); Hentschel, Klaus, Dr., 63517 Rodenbach (DE); Möller, Rolf, 64832 Rodenbach (DE); Ehrhardt, Knut, 63454 Hahau (DE)

(57) **Zusammenfassung**

Die Erfindung betrifft eine neue Produktform für Cyanurchlorid, nämlich Formlinge, insbesonders Pastillen, Schuppen und Stranglinge. Die Formlinge weisen gegenüber Pulver Vorteile bei der Handhabung auf.

Die Formlinge lassen sich herstellen durch tropfen- oder streifenförmiges Aufbringen von geschmolzenem Cyanurchlorid auf eine Fläche und Abführen der Schmelzwärme durch Kühlen der Fläche oder Kontaktieren der tropfen- oder streifenförmig aufgebrachten Schmelze mit einem Kühlgas.

## Beschreibung

Die Erfindung betrifft eine neue Produktform von festem Cyanurchlorid, nämlich Formlinge, insbesondere solche in Pastillen- oder Schuppenform. Ein weiterer Gegenstand betrifft ein Verfahren zur Herstellung von Cyanurchlorid-Formlingen, insbesondere Pastillen und Schuppen sowie Stranglinge.

Es ist bekannt, durch Trimerisation von Chlorcyan gewonnenes dampfförmiges Cyanurchlorid direkt oder über flüssiges Cyanurchlorid in festes Cyanurchlorid in feinteiliger Form zu überführen:

Die Abscheidung von pulverförmigem Cyanurchlorid durch Desublimation von dampfförmigem Cyanurchlorid kann in von außen gekühlten Räumen erfolgen oder durch Einleiten des Cyanurchloriddampfes mit einem Inertgas und/oder einer bei dem Abscheidevorgang verdampfenden indifferenten Kühlflüssigkeit in eine Abscheidekammer - siehe beispielsweise DE-PS 12 66 308 und US-PS 4,591,493. Bei der Gewinnung von feinteiligem Cyanurchlorid aus flüssigem Cyanurchlorid wird letzteres in eine Abscheidekammer eingedüst und mit im Kreis geführten inerten Kühlgasen der durch indirekte Kühlung in der Abscheidekammer abgekühlt, bis sich die Sprühtröpfchen in kristalliner Form abscheiden - siehe beispielsweise DE 28 43 379. Gemeinsam ist den Verfahren ein erheblicher technischer Aufwand für Abscheidekammern und Vorrichtungen zum Rückführen und Reinigen von Prozeß- und Abgasen.

Bei den zuvor gewürdigten sowie auf den gleichen Prinzipien beruhenden Verfahren wird Cyanurchlorid stets in feinteiliger Form, im allgemeinen mit einem maximalen Korndurchmesser von im wesentlichen kleiner 250 µm, erhalten. Derart feinteilige Produkte sind zwar vorteilhaft hinsichtlich ihrer hohen Reaktivität, sie weisen aber eine Reihe von Nachteilen auf, welche für viele Zwecke eine andere Produktform wünschenswert machen.

Die Handhabung, wie Förderung, Lagerung und Dosierung, von feinteiligem Cyanurchlorid bereitet besondere Probleme, weil zur üblichen Staubbildung feinteiliger Stoffe, welche Absaugvorrichtungen erforderlich machen, die korrosiven und reizenden Eigenschaften kommen. Zusätzlich ist Cyanurchlorid hydrolyseempfindlich, wobei dabei gebildete Hydrolyseprodukte nicht nur Cyanurchlorid selbst, sondern auch daraus hergestellte Folgeprodukte verunreinigen können. Feinteiliges Cyanurchlorid ist wegen seiner hohen Oberfläche der Hydrolyse besonders stark zugänglich. Damit kommt es auch leicht zu festen Ablagerungen in den Entstaubungsvorrichtungen und staubführenden Leitungen. Zur Vermeidung und Behebung aufgetretener Störungen sind technisch aufwendige Maßnahmen beziehungsweise Einrichtungen nötig.

Ein weiterer Nachteil feinteiligen Cyanurchlorids ist die unbefriedigende Rieselfähigkeit. Letztere läßt sich zwar durch die Zumischung von Rieselhilfsmitteln, etwa Kieselsäuren, verbessern, jedoch mindert das Rieselhilfsmittel die Produktreinheit des Cyanurchlorids und gegebenenfalls auch der daraus hergestellten Produkte. Gemäß EP-A 0 416 584 läßt sich die Fließfähigkeit von durch Desublimation oder Sprühkristallisation hergestelltem festen Cyanurchlorid auch ohne Zugabe eines Rieselhilfsmittels durch eine Scherbehandlung desselben in einem Kneter oder Mischer, insbesondere bei 60 bis 120 °C, verbessern; die Feinpulvrigkeit des Cyanurchlorids wird aber bei diesem Verfahren nicht beseitigt, denn die mittlere Korngröße beispielhafter Ausführungsformen liegt im Bereich von etwa 10 bis 40 µm.

Außer in feinteiliger Form kommt Cyanurchlorid auch in flüssiger Form in den Handel. Die Gewinnung von Cyanurchlorid in flüssiger Form ist beispielsweise aus der DE-PS 23 32 636 bekannt. Die flüssige Cyanurchlorid-Produktform erfordert aber auch über den Schmelzpunkt von Cyanurchlorid heizbare Lager- und Transportbehälter. Derartige Einrichtungen sind zwar für Verwender mit einem großen und regelmäßigen Bedarf wirtschaftlich, nicht aber für Verwender mit einem kleineren und/oder unregelmäßigen Bedarf.

Aufgabe der vorliegenden Erfindung ist demgemäß, eine neue feste Produktform für Cyanurchlorid aufzuzeigen, welche die Nachteile des feinteiligen Cyanurchlorids zumindest in deutlich geringerem Umfang aufweist. Insbesondere sollte die neue Produktform einfacher handhabbar sein, um Betriebsstörungen und/oder arbeitshygienische Probleme zu mindern.

Die Aufgabe wird gelöst durch Cyanurchlorid-Formlinge, insbesondere Pastillen, Schuppen und Stranglinge. Zweckmäßigerweise liegt die Dicke von Pastillen und Schuppen im Bereich von 0,5 bis 3 mm, wobei aber dickere oder dünnere Formlinge nicht ausgeschlossen werden. Vorzugsweise sind Schuppen zwischen etwa 0,5 und 2 mm und Pastillen zwischen 1 und 3 mm dick. Der Durchmesser ellipsoidförmiger Pastillen liegt vorzugsweise im Bereich von 2 bis 10 mm, insbesondere 3 bis 6 mm. Bei den Schuppen handelt es sich um flächige Formlinge der genannten Dicke, etwa streifenförmige Schuppen mit einer Breite zwischen 5 und 10 mm und einer Länge zwischen 10 und 50 mm oder um unregelmäßig gebrochene Schuppen ähnlicher Größe. Stranglinge haben im allgemeinen einen Durchmesser von 1 bis 10 mm, insbesondere 2 bis 5 mm, und eine Länge zwischen 10 und 50 mm. Bevorzugte Formlinge sind im wesentlichen frei von staubförmigem Cyanurchlorid. Unter 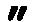 im wesentlichen frei 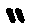 wird verstanden, daß eine geringe Menge Staub, vorzugsweise weniger als 5 Gew.-%, insbesondere weniger als 2 Gew.-%, entstanden durch Abrieb der Formlinge und/oder durch auf der Oberfläche der Formlinge aufsublimiertes Cyanurchlorid, nicht ausgeschlossen wird.

Die erfindungsgemäße Produktform läßt sich problemlos, das heißt, im wesentlichen ohne Staubentwicklung absacken und aus Gebinden entleeren. Bei der Förderung innerhalb eines Produktionsbetriebes kommt es nicht mehr zu gegebenenfalls nur mühsam zu beseitigenden Ablagerungen und Verstopfungen von Rohrleitungen. Durch die gegenüber feinteiligem Cyanurchlorid geringere Oberfläche sind die Verbackungs- und die Hydrolysegefahr wesentlich gemindert. Die erfindungsgemäßen Formlinge zeichnen sich gegenüber pulverförmigem Cyanurchlorid auch durch eine höhere Reinheit aus, da der Hydrolysatgehalt geringer ist und Rieselhilfsmittel entbehrlich sind. Obgleich die Nachteile von feinteiligem Cyanurchlorid lange bekannt waren und ein erheblicher technischer Aufwand nötig war, um trotz der Nachteile das Produkt sicher verarbeiten zu können, war es überraschend, daß die erfindungsgemäßen Formlinge, insbesondere Pastillen und Schuppen, bisher nie als Produktform für Cyanurchlorid in Betracht gezogen wurden.

Ein Verfahren zur Herstellung erfindungsgemäßer Cyanurchlorid-Formlinge, insbesondere Pastillen und Schuppen, umfaßt tropfenförmiges oder streifenförmiges Aufbringen von Cyanurchlorid in geschmolzender Form auf eine Fläche, Abführen der Schmelzwärme des Cyanurchlorids durch Kühlung der Fläche oder/und Kontaktieren der auf die Fläche aufgebrachten Schmelze mit einem Kühlgas und Abnehmen der erstarrten Formlinge von der Fläche.

Die Fläche, auf welche die Cyanurchloridschmelze aufgebracht wird, kann in beliebiger Weise gestaltet sein. Es kann sich um eine von der Rückseite gekühlte glatte oder strukturierte, vorzugsweise glatte, Fläche handeln. Die Fläche kann eben oder in Form einer Walze ausgebildet sein. Bei stationärer Anordnung einer ebenen gekühlten Fläche sind zusätzlich Vorrichtungen zum Abnehmen der Formlinge von der Fläche nötig, beispielsweise rakelartige Abnehmer. Zur Kühlung der Rückseite der Fläche kommen an sich bekannte Einrichtungen zur Anwendung, insbesondere Überströmen oder Besprühen der Rückseite der Fläche mit einem flüssigen oder gasförmigen Medium. Anstelle der Abfuhr der Schmelzwärme durch die Fläche ist es auch möglich, diese mittels eines gegenüber Cyanidchlorid inerten Kühlgases, wie gekühlte Luft oder Stickstoff, mit welchem die auf der Fläche aufgetragenen Schmelze kontaktiert wird, abzuführen. Bei der letztgenannten Ausführungsform kann z. B. ein Kühlgas direkt auf die Fläche geleitet werden, oder die Fläche wird durch einen gekühlten Raum transportiert; bei der Fläche kann es sich hierbei zum Beispiel um ein Transportband handeln.

Gemäß einer bevorzugten Ausführungsform des Verfahrens verwendet man als gekühlte Fläche ein endloses Kühlband, üblicherweise aus einem metallischen, gegenüber Cyanurchlorid korrosionsbeständigen Werkstoff. Das Kühlband kann von der Rückseite mittels eines Kühlmediums durch Besprühen oder Überströmen gekühlt werden. Bevorzugte Kühlmedien sind Wasser und Glykole oder Kühlgase. Auch eine indirekte Kühlung, etwa durch Leiten des Kühlbands über eine gekühlte Fläche, etwa innengekühlte Walzen oder Gleittische, ist möglich. Vorrichtungen mit einem Kühlband sind bekannt und im Handel erhältlich. Mit einer derartigen Vorrichtung kann das Verfahren kontinuierlich betrieben werden.

Die Cyanurchloridschmelze wird vorzugsweise mit einer Temperatur im Bereich von 150 bis 190 °C unter Verwendung geeigneter Auftragsvorrichtungen tropfenförmig oder streifenförmig oder in Form eines breiteren Bandes auf die Fläche, beispielsweise eine Kühlwalze oder Kühlband oder ein durch einen Kühlraum befördertes Band aufgebracht, wo die Tropfen in Form von Pastillen und die Streifen als flache Formlinge erstarren. Endlose Streifen können in einfacher Weise gebrochen werden, wobei Schuppen mit unregelmäßiger Form erhalten werden.

Der Durchmesser der Austrittsöffnungen der Auftragsvorrichtungen für die Schmelze bestimmt die Größe der Tropfen beziehungsweise die Breite des Strahls und damit diejenige der Pastillen beziehungsweise die Breite der Streifen. Zur Erzeugung von Pastillen oder streifenförmigen Formlingen werden vorzugsweise Austrittsbohrungen mit einem Durchmesser zwischen 1 und 3 mm verwendet oder Austrittsschlitze mit einer ähnlichen Weite und einer Breite, die etwa der gewünschten Streifenbreite entspricht. Durch Brechen der erstarrten Streifen lassen sich Schuppen mit einer Höhe von zum Beispiel 0,5 bis 5 mm, insbesondere 0,5 bis 2 mm, und einer Fläche von etwa 10 bis 500 mm², insbesondere 50 bis 200 mm².

Die Oberflächenspannung des Cyanurchlorids läßt die Herstellung eines stabilen Tropfens Zu. Aufgrund der geringen Viskosität des flüssigen Cyanurchlorids tritt bis zum vollständigen Erstarren eine Abflachung der Tropfen ein, die zu ellipsoidförmigen Pastillen führt. Zur Aufbringung von Tropfen eignen sich mittels hydrostatischem Druck belastete Auftragsvorrichtungen, beispielsweise Düsen mit einem Durchmesser zwischen etwa 1 und 3 mm. Eine gezielte Dosierung der Cyanurchloridschmelze ist auch unter Verwendung von Dosierpumpen und Düsen mit einem Durchmesser zwischen 0,5 und 2 mm möglich. Bei größeren Düsendurchmessern ist aufgrund des größeren Tropfens auch die flächenspezifisch abzuführende Wärme größer. Die dadurch bedingte längere Erstarrungszeit führt zu einem längeren Fließen des Cyanurchlorids, wodurch flachere Pastillen entstehen. Da beim Streifengießen keine Zwischenräume auf dem Kühlband entstehen, tritt ein Fließen nur am Rand des Streifens auf; nachdosiertes Cyanurchlorid staut sich auf den unteren Cyanurchloridschichten auf, und hierdurch sind dickere Schichten erhältlich. Sowohl Pastillen als auch Streifen erstarren innerhalb weniger Sekunden - ca. 15 s zur Abkühlung von 145 auf 35 °C - und lassen sich, ohne daß es zu Anklebungen kommt, gut von der gekühlten Fläche, etwa einem Kühlband, lösen. Die Cyanurchlorid-Pastillen beziehungsweise streifenförmigen Schuppen kristallisieren während des Erstarrens vollständig durch.

Die Figur zeigt schematisch eine zweckmäßige Vorrichtung zur Erzeugung von Pastillen und streifenförmigen Schuppen:

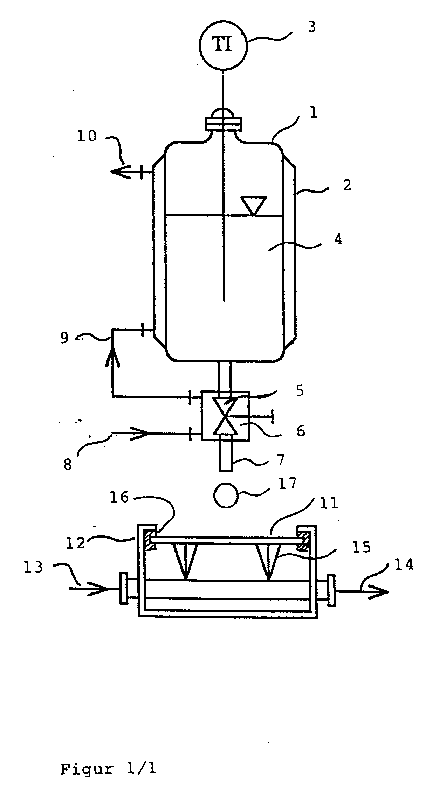

Im Behälter (1) mit einem Heizmantel (2) und Temperaturmeßvorrichtung (3) befindet sich die Cyanurchloridschmelze (4). Die Schmelze gelangt aus dem Behälter über ein Dosierventil (5) mit Heizmantel (6) zur Düse (7). Über Leitung (8) wird ein Wärmeträgermedium zugeführt; über die Leitung (9) gelangt das Wärmeträgermedium von dem heizbaren Dosierventil zum heizbaren Behälter, über Leitung (10) wird es abgeführt. Die Kühlbandvorrichtung - die Figur zeigt diese im Schnitt quer zur Transportrichtung des Bandes - umfaßt das Kühlband (11), ein Gehäuse (12) für die Aufnahme des Kühlmediums sowie eine Zu- und Abführleitung (13 und 14) für dieses. Zwischen Gehäuse und Kühlband befinden sich geeignete Dichtelemente (16), um einen Kontakt zwischen Cyanurchloriddampf und Dämpfen des Kühlmediums, etwa Wasserdampf, zu vermeiden. Die Antriebsvorrichtung für das Kühlband und seine Rückführung sind in Figur 1 nicht dargestellt. Die aus der Düse austretenden Tropfen (17) fallen auf das Kühlband und erstarren dort. Die Länge und Abzugsgeschwindigkeit des Kühlbands werden so dimensioniert, daß eine vollständige Erstarrung der aufgetragenen Schmelze möglich ist. Bei der dargestellten Ausführungsform wird das Kühlmedium auf die Rückseite des Kühlbands mit dem Kühlmedium besprüht (15).

Gemäß einer bevorzugten Ausführungsform befindet sich die Auftragsfläche für die Schmelze in einem mit einer Abgasaufbereitung verbundenen geschlossenen Raum, dessen der Auftragsfläche gegenüberliegende Fläche auf eine Temperatur oberhalb der Desublimationstemperatur des Cyanurchlorids geheizt wird und/oder in welchem ein Inertgasstrom, etwa Stickstoff, über die Auftragsfläche geleitet wird, um Sublimat mit dem Gas auszutragen.

Gemäß einer weiteren Ausführungsform lassen sich Cyanurchlorid-Formlinge als Stranglinge mit einer Länge von vorzugsweise im Bereich von 10 bis 50 mm und einer Dicke von vorzugsweise 1 bis 10 mm, insbesondere 3 bis 5 mm, erzeugen. Hierbei wird flüssiges Cyanurchlorid mit einer Temperatur wenige °C oberhalb der Schmelztemperatur, zweckmäßigerweise um 160 °C, in eine Kapillare geleitet. Zur Auskristallisierung wird die Schmelzwärme über die Kapillarwand abgeführt; zudem wird der Feststoffstrang in der Kapillare nachfolgend weiter unterkühlt, vorzugsweise auf um/unter 50 °C, um eine Sublimation nach Feststoffaustrag aus der Kapillare zu vermindern.

Cyanurchlorid liegt in flüssiger und fester Phase in der Kapillare im T-Bereich von etwa 160 °C bis etwa 50 °C so vor, daß keine Verdampfung beziehungsweise Sublimation möglich ist. Während des Erstarrens und der Abkühlung kommt es zu keiner Gasentwicklung und damit zu keiner Staubentstehung durch Desublimat. Der kompakte Strang definierten Durchmessers läßt sich gut in Stranglinge definierter Länge zerteilen.

Die Stranglinge lassen sich beispielsweise in einer üblichen, im Druckzellenbereich kühlbaren, Strangpresse herstellen - siehe Beispiel 2. Alternativ hierzu ist es auch möglich, Cyanurchloridschmelze in dünnen PTFE-Schläuchen erstarren zu lassen und den Strang vor/nach Entfernen des Schlauchs in die Stranglinge zu zerteilen.

Das Verfahren läßt sich einfach durchführen. Die erforderlichen Vorrichtungen zur Durchführung des Verfahrens erfordern eine geringere Investitionssumme als sie für herkömmliche Abscheidekammern mit den erforderlichen zusätzlichen Einrichtungen zum Versprühen einer Cyanurchloridschmelze oder zur Desublimation von Cyanurchloriddampf nötig ist. Die bisher durch Staub und Betriebsstörungen verursachte Ausbeuteminderung bei den vorbekannten Verfahren zur Herstellung von feinteiligem Cyanurchlorid entfällt beim erfindungsgemäßen Verfahren.

Die verfahrensgemäß erhältlichen Cyanurchlorid-Formlinge zeichnen sich dadurch aus, daß bisher unvermeidliche arbeitshygienische Probleme entfallen und daß die Neigung zur Verbackung und die Gefahr der Verunreinigung durch Hydrolysatbildung gemindert wurden.

Die erfindungsgemäßen Cyanurchlorid-Formlinge lassen sich wie feinteiliges Cyanurchlorid zur Herstellung von Cyanurchlorid-Folgeprodukten verwenden. Die Herstellung der Folgeprodukte kann in organischer oder wäßriger Phase oder in zweiphasigen Lösungsmittelsystemen erfolgen. Bei Umsetzungen in suspendiertem Zustand kann es zweckmäßig sein, die Formlinge innerhalb des Reaktionsmediums zu zerkleinern, etwa mittels einer Naßmühle.

### Beispiel 1

In der in der Figur beschriebenen Anlage wurden Cyanurchlorid-Pastillen und streifenförmige Schuppen hergestellt. Das verwendete Kühlband wurde mit Wasser (15 °C) gekühlt. Der Bandvorschub betrug 3 m/min. Die Temperatur der Cyanurchloridschmelze betrug 182 bis 183 °C. Unter Verwendung einer Düse mit einem Durchmesser von 3 mm war eine kontinuierliche Tropfenbildung bei leichter Überhitzung der Schmelze möglich. Erhalten werden Pastillen mit einem nur geringfügig streuenden Durchmesser von etwa 4 mm (± 0,2) und einer Höhe von etwa 1,5 mm. Die Tabelle zeigt einige Stoffdaten der erfindungsgemäßen Pastillen im Vergleich zu einem handelsüblichen feinteiligen Cyanurchlorid (Qualität F der Degussa AG).

**Tabelle**

| | Pastillen | Cyanurchlorid Qualität F |
|---|---|---|
| Hydrolysatgehalt | 0,21 % | 0,32 % |
| SiO₂-Gehalt | 0 % | 0,14 % |

Die Bestimmung des Hydrolysatgehalts basiert darauf, daß Hydrolyseprodukte des Cyanurchlorids nicht in Toluol löslich sind und sich nach Auflösung des Cyanurchlorids in Toluol leicht abtrennen und gravimetrisch bestimmen lassen.

### Beispiel 2

Hergestellt wurden Strangpresslinge unter Verwendung einer Strangpresse, umfassend eine Druckzelle mit einer Kapillare (Durchmesser: 5 mm), eine mittels einer Spindelpresse betreibbare Drucknadel und einen Druckzellenfuß zur Aufnahme und Abführung des Strangs. Die Druckzelle wurde durch Anblasen mit Stickstoff gekühlt. Die Druckzelle wurde mit flüssigem (T = 160 °C) Cyanurchlorid befüllt; durch Anblasen der Zelle kühlte der Zelleninhalt unter Kristallisation des Cyanurchlorids ab. Die Temperatur des Strangs am Austritt lag bei etwa 50 °C. Durch Krafteinwirkung auf die Drucknadel wurde ein Cyanurchlorid-Strang ausgetragen, der Strang in 15 bis 50 mm lange Stücke zerteilt. Die erhaltenen Strangpresslinge sind staubfrei und lager- und transportfähig.

## Patentansprüche

1. Cyanurchlorid-Formlinge, insbesondere Pastillen, Schuppen und Stranglinge.

2. Cyanurchlorid nach Anspruch 1,
dadurch gekennzeichnet,
daß die Dicke der Pastillen oder Schuppen im Bereich von 0,5 bis 3 mm und der Durchmesser der Stranglinge im Bereich von 1 bis 10 mm liegt.

3. Cyanurchlorid nach Anspruch 2,
dadurch gekennzeichnet,
daß die Pastillen einen Durchmesser im Bereich von 2 bis 10 mm und die Schuppen eine Fläche im Bereich von 10 bis 500 mm² aufweisen.

4. Verfahren zur Herstellung von Cyanurchlorid-Formlingen, insbesondere Pastillen und Schuppen, gemäß einem der Ansprüche 1 bis 3,
umfassend tropfenförmiges oder streifenförmiges Aufbringen von Cyanurchlorid in geschmolzender Form auf eine Fläche, Abführen der Schmelzwärme des Cyanurchlorids durch Kühlung der Fläche oder/und Kontaktieren der auf die Fläche aufgebrachten Schmelze mit einem Kühlgas und Abnehmen der erstarrten Formlinge von der Fläche.

5. Verfahren nach Anspruch 4,
dadurch gekennzeichnet,
daß man es unter Verwendung eines Kühlbandes aus einem metallischen Werkstoff kontinuierlich durchführt, wobei die Schmelze mittels eines mit der Rückseite des Kühlbands in Kontakt gebrachten Kühlmediums, insbesondere Kühlwasser, abgeführt wird.

6. Verfahren nach Anspruch 4 oder 5,
dadurch gekennzeichnet,
daß man eine Cyanurchloridschmelze mit einer Temperatur im Bereich von 150 bis 190 °C, insbesondere 170 bis 190 °C, unter Verwendung von tropfen- oder strahlbildenden Einrichtungen mit einem Durchmesser im Bereich von 1 bis 3 mm tropfenförmig oder streifenförmig auf eine gekühlte Fläche aufträgt.

7. Verfahren zur Herstellung von Cyanurchlorid-Stranglingen, umfassend Befüllen einer mit einer Kapillare versehenen Zelle eines Strangwerkzeugs, insbesondere einer Druckzelle einer Strangpresse, mit Cyanurchloridschmelze mit einer Temperatur von 1 bis 20 °C oberhalb des Schmelzpunkts, Abkühlen der Zelle zwecks Kristallisation des Cyanurchlorids in der Kapillare auf eine Temperatur im Bereich von unterhalb des Schmelzpunkts bis etwa 50 °C und Auspressen des Strangs mittels Krafteinwirkung auf eine Drucknadel.

8. Verwendung von Cyanurchlorid-Formlingen, insbesondere Pastillen, Schuppen und Stranglinge, zur Herstellung von Cyanurchloridderivaten.
